# EUROPEAN PATENT APPLICATION

(11) **EP 3 026 390 A1**
(43) Date of publication of application: **01.06.2016**
(21) Application number: 15192032.9
(22) Date of filing: 29.10.2015
(51) Int. Cl.: G01B 9/02, A61B 5/00

(54) **INTERFEROMETRIC BIOLOGICAL MEASUREMENT DEVICE AND BIOLOGICAL MEASUREMENT METHOD**

(30) Priority: 26.11.2014 JP 2014238887; 25.06.2015 JP 2015127160
(71) Applicant: Panasonic Intellectual Property Management Co., Ltd., Osaka-shi, Osaka 540-6207 (JP)
(72) Inventor: KABETANI, Yasuhiro, Osaka 540-6207 (JP); OIKAZE, Hirotoshi, Osaka 540-6207 (JP); TAKECHI, Yohei, Osaka 540-6207 (JP); FURUTA, Tomotaka, Osaka 540-6207 (JP)
(74) Representative: Vigand, Philippe

(57) **Abstract**

A biological measurement device includes a phase modulation type interferometer, a first stage, a light transmitting plate, and a second stage. The first stage is able to retain a target which is a biological body. The light transmitting plate is disposed between the first stage and the interferometer in a direction of an optical axis of measurement light of the interferometer. The second stage supports the light transmitting plate. The first stage is configured to expand by a pressure of a gaseous body in the direction of the optical axis of the measurement light of the interferometer and to press the target against the light transmitting plate.

## Description

### TECHNICAL FIELD

The present invention relates to a biological measurement device which measures a biological body by using an interferometer, and a method thereof.

### BACKGROUND ART

In the related art, a white interferometer is used for precisely measuring a microscopic distance in an industrial field. Among such white interferometers, a phase modulation type interference microscope is known. According to this interference microscope, a phase modulation of the degree of a wavelength of light is applied to a difference in an optical path length between measurement light and reference light, and thus a cross-section of an analysis target in a direction horizontal to an optical axis of the analysis target is measured. In such a microscope, it is possible to measure an XY cross-section having a depth at which the optical path lengths are coincident with each other (refer to Published Japanese Translation No. 2004-528586 of the PCT International Publication).

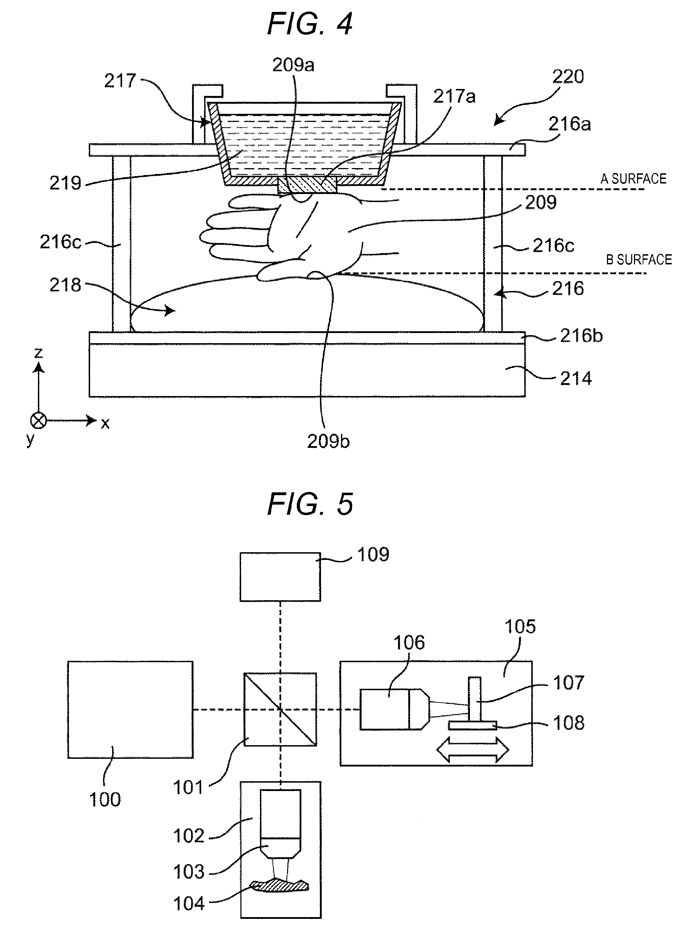

FIG. 5 illustrates an optical interference measurement device using a Linnik interferometer. Light emitted from light source 100 enters beam splitter 101, and the split lights are transmitted to arms 102 and 105 of an optical interferometer. In first arm 102, lens 103 and analysis target 104 are disposed. In second arm 105, lens 106 and reference surface 107 are disposed. On reference surface 107, as a phase modulation mechanism, a driving mechanism formed of PZT element 108 is disposed. Such a driving mechanism is capable of accurately moving reference surface 107 even in a microscopic movement distance of nm order which is a wavelength of the light. The lights transmitted to arms 102 and 105 are reflected from analysis target 104 and reference surface 107, respectively. The reflected lights enter camera 109 through beam splitter 101.

In such a microscope, analysis target 104 is scanned in XY directions and is repeatedly measured, and thus a broad XY cross-section of analysis target 104 can be measured. Furthermore, in the interferometer described above, a phase is continuously subjected to a sine modulation.

### SUMMARY OF THE INVENTION

The present invention provides a biological measurement device and a biological measurement method which reduce an influence of pulsation at the time of performing biological measurement.

The biological measurement device according to the present invention includes a phase modulation type interferometer, a first stage, a light transmitting plate, and a second stage. The first stage is capable of retaining a target which is a biological body. The light transmitting plate is disposed between the first stage and the interferometer in an optical axis direction of measurement light of the interferometer. The second stage supports the light transmitting plate. The first stage is configured to expand by a pressure of a gaseous body in the optical axis direction of the measurement light of the interferometer and to press the target against the light transmitting plate.

In the biological measurement method according to the present invention, the target which is the biological body is measured by using the biological measurement device described above. In this biological measurement method, the target is retained with the first stage between the first stage and the light transmitting plate in the optical axis direction of the measurement light of the interferometer. Then, the target is measured while retained and pressed with the first stage against the light transmitting plate.

According to an aspect of the present invention, the target is pressed against the light transmitting plate and is subjected to biological measurement, and thus it is possible to reduce an influence of pulsation at the time of performing the biological measurement.

### BRIEF DESCRIPTION OF DRAWINGS

FIG. 1 is a schematic view of a biological measurement device in a state in which a biological body is measured according to an exemplary embodiment of the present invention.
FIG. 2 is a partial sectional view in which an upper plate and a solvent container of the biological measurement device illustrated in FIG. 1 are seen from a horizontal direction.
FIG. 3 is a flowchart illustrating a biological measurement method according to the exemplary embodiment of the present invention.
FIG. 4 is an explanatory view for a displacement of an analysis target according to the exemplary embodiment of the present invention.
FIG. 5 is a schematic view of an optical interference measurement device according to a method of the related art.

### DETAILED DESCRIPTION OF PREFERRED EMBODIMENTS

Prior to describing an exemplary embodiment of the present invention, problems of a measurement device of the related art will be simply described. When the measurement device of the related art is applied to measurement of a soft and pulsative biological body, it is not possible to get the analysis target still compared to a microscopic phase modulation of the degree of a wavelength of light. For this reason, a portion which is different from a measurement position may be measured.

Hereinafter, the exemplary embodiment of the present invention will be described with reference to the drawings.

### Optical Interferometer

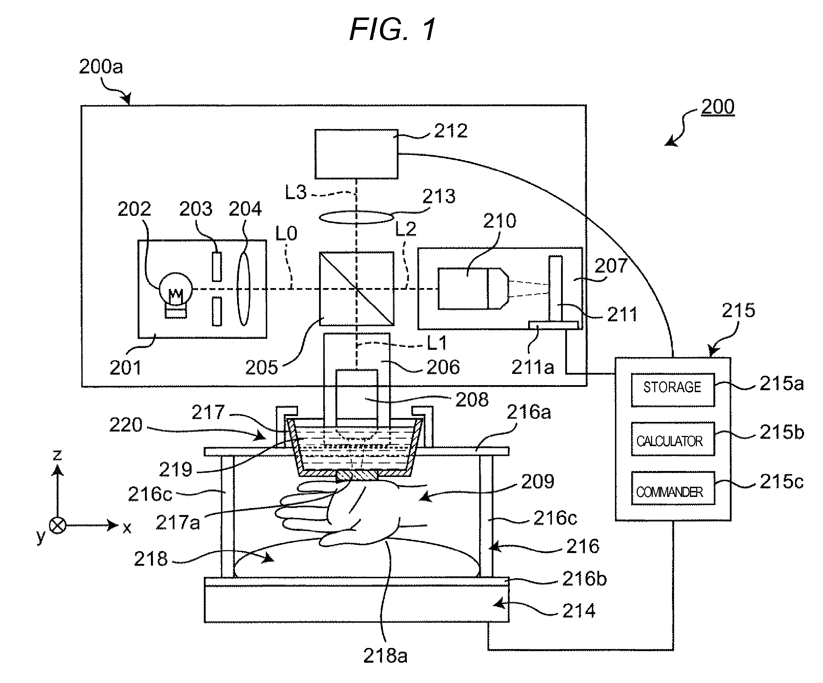

FIG. 1 is a schematic view of biological measurement device 200 according to the exemplary embodiment of the present invention. Biological measurement device 200 mainly includes phase modulation type Linnik interferometer 200a, first stage 218, and second stage 220. Biological measurement device 200 may further include mechanical stage 214. For example, second stage 220 is formed of gatepost type jig (solvent container support member) 216, solvent container 217, and mechanical stage 214, and supports optical window 217a.

Linnik interferometer 200a is an example of interferometers, and includes light source 201, beam splitter 205, first arm 206, second arm 207, imaging lens 213, camera 212, and controller 215.

Light source 201 is a unit capable of emitting light. Light source 201 includes lamp 202, diaphragm 203, and collimating lens 204. Lamp 202 is a light source emitting low coherence light, such as, for example, a halogen lamp, a xenon arc lamp, a mercury lamp, or an LED. A wavelength width Δλ of the low coherence light can be, for example, greater than 50 nm and less than 400 nm in order to increase depth resolution and to suppress a blurring of an image due to chromatic aberration. A center wavelength λc of the low coherence light can be, for example, greater than 20 nm and less than 1000 nm in order to obtain a high diffraction limit as a microscope.

Light L0 emitted from light source 201 enters beam splitter 205. Meanwhile, beam splitter 205 may be a cube type beam splitter or a plate type beam splitter. When beam splitter 205 is the cube type beam splitter, a cube surface may be inclined with respect to an optical axis in order to reduce the possibility that reflected light from the cube surface becomes stray light and causes signal noise.

Light L0 entered beam splitter 205 is separated into measurement light L1 and reference light L2 having amplitudes which are identical to each other. Measurement light L1 and reference light L2 are transmitted to first arm 206 and second arm 207 of the interferometer, respectively.

Measurement light L1 is transmitted to first arm 206. First objective lens 208 is disposed in first arm 206.

A focal surface of measurement light L1 which enters first objective lens 208 is set in analysis target (hereinafter, target) 209 by first objective lens 208. After causing Fresnel reflection and backward scattering on the focal surface, measurement light L1 returns to beam splitter 205 through first objective lens 208.

Reference light L2 is transmitted to second arm 207. Second objective lens 210 and reference surface 211 are disposed in second arm 207. A focal point of reference light L2 is set on reference surface 211 by second objective lens 210. After reflected on reference surface 211, reference light L2 returns to beam splitter 205.

At this time, when the optical path length of first arm 206 is coincident with the optical path length of second arm 207 in a range of coherence length Lc of light source 201, measurement light L1 and reference light L2 returning to beam splitter 205 cause optical interference. Imaging lens 213 disposed between beam splitter 205 and camera 212 allows light L3 in which the optical interference occurs due to measurement light L1 and reference light L2 returning to beam splitter 205 to form an image on camera 212 by focusing thereon.

Camera 212, for example, includes a sensor configured of a CCD type two-dimensional pixel. A sensor surface of camera 212 and target 209, and the sensor surface and reference surface 211 are respectively arranged to be in an optically conjugate relation. That is, camera 212 is capable of setting target 209 and reference surface 211 so as to overlap them in a viewing field thereof.

Furthermore, optical magnifications at the time of being in the conjugate relation may be different from each other insofar as the sensor surface and target 209, and the sensor surface and reference surface 211 are respectively conjugated. However, in order to reduce measurement error due to optical-system aberration, it is preferable that the magnifications are same. In addition, in order to reduce a measurement error due to wavelength dispersion, it is more preferable that the same glass material is used.

Controller 215 is composed of a computer. Controller 215 includes storage 215a, calculator 215b, and commander 215c. Storage 215a stores data acquired by camera 212. Calculator 215b processes an image of target 209. Commander 215c outputs an operation command to each of a shutter of camera 212 and mechanical stage 214. The shutter of camera 212 and mechanical stage 214 are capable of being synchronously driven through controller 215. The shutter of camera 212 may be an electronic shutter, or a mechanical shutter may be disposed on an entire surface of a light receptor.

Reference surface 211 is a mirror, and is driven by driving mechanism 211a configured, for example, of a PZT element under the control of controller 215 and modulates the optical path length. In addition, storage 215a, calculator 215b, and commander 215c may be processors disposed on a common circuit board, or may be separate devices. Then, a predetermined command stored in these processors or devices is executed.

Mechanical stage 214 is a triaxial stage, and is capable of independently scanning in X, Y, and Z directions which are orthogonal to each other. Gatepost type jig 216 is disposed on mechanical stage 214.

Jig 216 includes upper plate 216a, lower plate 216b, and a plurality of columnar supports 216c connecting upper plate 216a to lower plate 216b. Columnar supports 216c, for example, are four columns in total which are erected at each corner when upper plate 216a and lower plate 216b are square plates. Solvent container 217 is fixed to the center of upper plate 216a. First stage 218 is disposed on an upper surface of lower plate 216b.

An upper portion of solvent container 217 is opened to face first objective lens 208 of first arm 206. Solvent container 217 is provided with optical window 217a having a flat lower surface on a bottom surface thereof. Optical window 217a is capable of transmitting measurement light L1 from first objective lens 208. Optical window 217a functions as an example of a light transmitting plate capable of transmitting measurement light L1.

Solvent container 217 is filled with medium 219 suitable for first objective lens 208 to be used. For example, when first objective lens 208 is a water immersion objective lens, medium 219 is water. When first objective lens 208 is an oil immersion objective lens, medium 219 is matching oil. When the biological body is measured, matching is performed by using silicone oil having the same refraction index as that of the biological body, and thus it is preferable that first objective lens 208 is a silicone oil immersion objective lens, and silicone oil is used as medium 219.

First stage 218 is capable of retaining target 209 in upper surface 218a and of driving target 209 in the Z direction (to move target 209 in the optical axis direction of measurement light L1). Specifically, first stage 218, for example, is a balloon which is stage-driven (moved in up and down directions) by applied with an air pressure. A lower surface of the balloon is fixed to lower plate 216b. Only upper surface 218a of the balloon is lifted up in the Z direction, and target 209 is lifted up by the balloon.

A stroke in the Z direction is sufficiently large such that target 209 can be set on upper surface 218a of first stage 218 before first stage 218 is lifted up. The stroke in the Z direction, for example, is 100 mm or more and 200 mm or less.

Both of solvent container 217 and first stage 218 are integrated with mechanical stage 214 through jig 216, and does not independently move. When mechanical stage 214 performs scanning in the X, Y, or Z direction, jig 216, solvent container 217, and first stage 218 are moved in the X, Y, or Z direction integrally with mechanical stage 214. That is, the scanning in the X, Y, or Z direction of mechanical stage 214 is identical to scanning in the X, Y, or Z direction of jig 216, solvent container 217, and first stage 218.

On the other hand, a portion of biological measurement device 200 excluding solvent container 217, first stage 218, jig 216, and mechanical stage 214 is separated from mechanical stage 214, and thus does not move even when mechanical stage 214 performs the scanning. Therefore, when mechanical stage 214 performs the scanning, a position of solvent container 217 with respect to first objective lens 208 is scanned. In other words, an opening is formed in the upper portion of solvent container 217 in a range in which first objective lens 208 can relatively perform scanning in the X and Y directions.

Alternatively, it is possible that the portion of biological measurement device 200 excluding solvent container 217, first stage 218, jig 216, and mechanical stage 214 is made to be capable of scanning in the X, Y, and Z directions, and mechanical stage 214 is fixed.

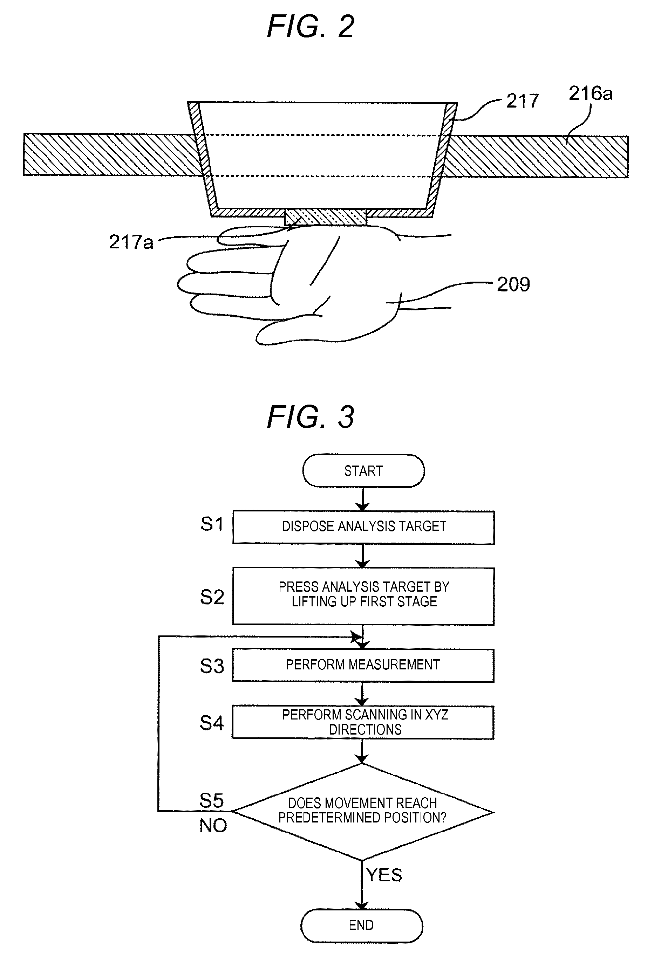

FIG. 2 is a partial sectional view illustrating upper plate 216a, solvent container 217, and optical window 217a. Optical window 217a protrudes from a lower surface of upper plate 216a toward a lower side in the Z direction (in the vicinity of first stage 218). Accordingly, target 209 can be contacted tightly to optical window 217a without a gap between target 209 and optical window 217a. When optical window 217a does not protrude from the lower surface of upper plate 216a toward the lower side in the Z direction, a gap is generated between target 209 and optical window 217a, and thus sufficient contact cannot be established, and the accuracy of the acquired image may decrease.

### Biological Measurement Method according to the Exemplary Embodiment

FIG. 3 is a flowchart illustrating a biological measurement method of the present exemplary embodiment. The biological measurement method of the present exemplary embodiment will be described with reference to FIG. 1 and FIG. 3.

First, in S1, target 209 is disposed. As an example, a human body is adopted as target 209 which is the biological body. More specifically, target 209 is a cell configuring the skin of a hand of the human body.

Target 209 is mounted on upper surface 218a of first stage 218 between optical window 217a and first stage 218. At this time, first stage 218 is at the lowest point in the Z direction, and a sufficient clearance is disposed such that target 209 can be mounted between upper surface 218a of first stage 218 and optical window 217a. That is, target 209 is retained with first stage 218 between first stage 218 and optical window 217a in the direction of the optical axis of the measurement light of interferometer 200a.

Next, in S2, upper surface 218a of first stage 218 is lifted up, and target 209 is pressed against optical window 217a. Accordingly, a lower surface of target 209 is compressed by upper surface 218a of first stage 218, and an upper surface of target 209 is compressed by optical window 217a. Thus, target 209 is compressed from each of the up and down directions.

Next, in S3, target 209 is measured. Specifically, commander 215c drives the shutter of camera 212, and thus camera 212 acquires images. Calculator 215b calculates an XY tomographic image from an interference signal which is obtained from target 209 among the images acquired by camera 212. The XY tomographic image is calculated by performing a phase modulation on reference surface 211 by using driving mechanism 211a of the PZT element.

Next, in S4, the scanning is performed in the X, Y, and Z directions. Mechanical stage 214 is driven by commander 215c, and solvent container 217, first stage 218, and the like are integrally moved. The XY tomographic image is obtained in S3, and thus the scanning is performed in the Z direction at the time of obtaining data in the Z direction, and the scanning is performed in the XY directions at the time of obtaining data in a wider range.

Next, in S5, controller 215 determines whether the movement of mechanical stage 214 in S4 reaches a predetermined position or not. When controller 215 determines that the movement reaches the predetermined position, the measurement ends. When controller 215 determines that the movement does not reach the predetermined position, the process returns to S3, and the series of measurements of S3 to S5 is repeatedly performed, and thus target 209 is moved towards the predetermined position.

### Flattening Contraption

In the present exemplary embodiment, first stage 218 is a balloon, and the shape thereof is changed according to a contact target, and thus even though the lower surface of target 209 is formed in any shape, first stage 218 can be tightly contacted to target 209. On the other hand, the upper surface of target 209 is pressed against the lower surface of optical window 217a, and thus the shape thereof is identical to that of the lower surface of optical window 217a, that is, is restrained into a planar shape. In other words, first stage 218 is capable of sandwiching target 209 together with optical window 217a therebetween. Therefore, the rigidity of the surface of first stage 218 where first stage 218 is in contact with target 209 is smaller than the rigidity of the lower surface of optical window 217a.

### Mechanism of Preventing Vibration from propagating to Window

FIG. 4 is a view illustrating a displacement of each portion with respect to that of target 209. First stage 218 expands by an air pressure in the optical axis direction of the measurement light of interferometer 200a illustrated in FIG. 1, and presses target 209 against optical window 217a. Accordingly, it is possible to prevent a shift in the position of target 209 with respect to the optical axis of the measurement light due to pulsation. This mechanism will be described as follows.

Assuming here a flat surface of target 209 which is in contact with optical window 217a in the Z direction as A surface 209a, and a flat surface which is in contact with first stage 218 in the Z direction as B surface 209b. When target 209 is the biological body, the volume thereof fluctuates due to biological activity such as pulsation and breathing. The fluctuation in the volume is divided into a fluctuation in the XY directions and a fluctuation in the Z direction.

In a case where a pressure due to the volume fluctuation is applied to each of optical window 217a, jig 216, and first stage 218, when A surface 209a fluctuates, the measurement position fluctuates in the Z direction. When the optical path length of the wavelength order of the phase modulation described above is changed due to the fluctuation in the Z direction, mutual cancellation occurs in the interference signal, and thus the interference signal is attenuated. For example, when the fluctuation in A surface 209a is 200 nm, the change in the optical path length is 400 nm in reciprocation of light, and light having a wavelength of 800 nm is completely cancelled. The fluctuation in A surface 209a causes cancellation of an interference pattern due to not only the fluctuation of 200 nm, but also a low coherence light source having a wide wavelength width.

For this reason, first stage 218 lifts up target 209 by the air pressure such that A surface 209a is not displaced, but only B surface 209b is displaced. Target 209 is lifted up by the air pressure, and thus it is possible to change (deform or displace) only B surface 209b according to the biological activity of target 209.

In order to displace only B surface 209b, it is more preferable that the rigidity of optical window 217a and jig 216 configuring second stage 220 is higher than the rigidity of first stage 218. For example, the displacement of A surface 209a is suppressed to be 10 nm or more and 100 nm or less such that the cancellation of the interference pattern does not occur, and the displacement of the first stage 218 is allowed to be approximately 1 mm at which the volume fluctuation in target 209 can be sufficiently absorbed.

Meanwhile, optical window 217a is formed of glass or quartz as an example. Jig 216 is formed of aluminum or iron as an example. Solvent container 217 supporting optical window 217a is formed of polycarbonate or acryl as an example.

According to the configuration described above, even when the volume of target 209 fluctuates according to the biological activity thereof, deformation of B surface 209b absorbs the influence. Therefore, the displacement of optical window 217a which is the measurement portion is suppressed, and thus the fluctuation in A surface 209a is suppressed, and thereby obtaining a signal with high accuracy, and it is possible to measure the biological body with high accuracy.

Meanwhile, when target 209 is the human body, a cycle of the pulsation is approximately 1 Hz. Accordingly, when the phase modulation can be performed sufficiently faster than the cycle of the pulsation, for example, at approximately 200 Hz, the mutual cancellation does not occur in the interference signal. However, in this case, a modulation speed of reference surface 211 is high, and thus a large load is applied to driving mechanism 211a such as the PZT element. For this reason, in the present exemplary embodiment, it is possible to measure the biological body with high accuracy without increasing the modulation speed of reference surface 211 and applying a large load to driving mechanism 211a.

### Mechanism of Determining Stage Pressure

In order to control a pressure which presses the upper surface of target 209 against optical window 217a, it is preferable that first stage 218 is driven by air pressure. If a mechanical stage using a motor, a ball screw mechanism, and the like in first stage 218 is used, the stroke in the Z direction becomes accurate compared to the balloon; however, the rigidity increases, and thus B surface 209b becomes rigid.

On the other hand, when first stage 218 is driven by the air pressure, it is possible to absorb the fluctuation in the volume of target 209 by B surface 209b, and it is possible to suppress the displacement of A surface 209a which is the measurement portion. It is preferable that the pressing pressure for absorbing the fluctuation by B surface 209b is sufficiently small so as not to exceed a blood pressure which is the pressure of the pulse of the human and is sufficiently large for regulating the position. When target 209 is the human body, as this range, the pressure is equal to or greater than a pressure suppressing the displacement of A surface 209a and equal to or less than the blood pressure, and specifically, a pressure of a gaseous body is 30 mmHg (3999.672 Pa) or more and 80 mmHg (10665.792 Pa) or less. In a case where target 209 is the human body, when the pressing pressure exceeds the pressure of the pulse of target 209, that is, the blood pressure, blood flow is blocked, and thus target 209 is adversely affected.

In order to prevent a positional shift due to compression in the XY directions which are in parallel with optical window 217a, it is preferable that the pressure is applied in the Z direction which is perpendicular to a surface of optical window 217a. At this time, the air pressure set such that optical window 217a and target 209 make a plane contact allows the position of target 209 to be rigidly regulated by a strong static friction force.

Meanwhile, when the pressure not only in the Z direction but also in the XY directions is applied to target 209 in order to fix target 209, the position of the target 209 in the XY directions is regulated, and thus a margin for escaping the fluctuation in the volume due to the pulsation is reduced. Therefore, it is preferable that the position of target 209 is not regulated in the XY directions. That is, it is preferable that first stage 218 is capable of retaining target 209 in a state that target 209 is opened in the XY directions.

In addition, it is preferable that a space between optical window 217a and target 209 is filled with medium 219. Medium 219 is capable of increasing a friction force between optical window 217a and target 209. At this time, it is preferable that optical performance of medium 219 is identical to imaging performance of an optical system. As medium 219, for example, fresh water, normal saline solution, skin toner, emulsion, oil, and silicone oil may be used.

Thus, according to the present exemplary embodiment, when the biological body is measured by using biological measurement device 200, it is possible to measure target 209 which is the biological body by pressing target 209 against optical window 217a, and it is possible to reduce the influence of the pulsation.

Meanwhile, in the above description, first stage 218 expands by the air pressure. However, it is not limited to air, and first stage 218 may expand by rare gas such as argon, or inert gas such as nitrogen gas. By using an inert gaseous body which rarely causes a chemical reaction as the gaseous body operating first stage 218, it is possible to suppress the influence on the measurement, and it is possible for an operator to safely perform an operation. That is, first stage 218 is configured to expand by the pressure of the gaseous body in the optical axis direction of the measurement light of interferometer 200a and to press target 209 against optical window 217a. In addition, when first stage 218 is formed of a balloon, the balloon expands by the pressure of the gaseous body with which the balloon is filled.

Meanwhile, from a viewpoint of maintainability or convenience, or from an object of observing the biological body in a more natural environment, it is most preferable that air is adopted as the gaseous body which allows first stage 218 to expand. More specifically, it is preferable to adopt air containing oxygen which has no harmful influence on the biological body even at the time of being leaked in large amounts, and specifically, it is more preferable to use air in which a content ratio of oxygen is identical to that of atmospheric air, which is 21 volume%.

Meanwhile, among various exemplary embodiments or modification examples described above, arbitrary exemplary embodiments or modification examples may be suitably combined, and thus it is possible to obtain an effect of each of the exemplary embodiments or modification examples. In addition, a combination of the exemplary embodiments, a combination of the examples, or a combination of the exemplary embodiments and the examples is available, and a combination of the characteristics of different exemplary embodiments or examples is also available.

As described above, the biological measurement device and the biological measurement method of the present invention can reduce the influence of the pulsation at the time of performing biological measurement. Therefore, they are useful at the time of performing biological measurement using the interferometer.

## Claims

1. A biological measurement device, comprising:
a phase modulation type interferometer;
a first stage capable of retaining a target which is a biological body;
a light transmitting plate disposed between the first stage and the interferometer in a direction of an optical axis of measurement light of the interferometer; and
a second stage supporting the light transmitting plate,
wherein the first stage is configured to expand by a pressure of a gaseous body in the direction of the optical axis of the measurement light of the interferometer, and to press the target against the light transmitting plate.

2. The biological measurement device according to claim 1,
wherein rigidity of the first stage is lower than rigidity of the second stage.

3. The biological measurement device according to any one of claims 1 and 2,
wherein the first stage is a balloon which expands by the gaseous body.

4. The biological measurement device according to any one of claims 1 to 3,
wherein the first stage retains the target in a state that the target is opened in a direction orthogonal to the optical axis of the measurement light of the interferometer.

5. The biological measurement device according to any one of claims 1 to 4,
wherein the light transmitting plate is an optical window capable of transmitting the measurement light from the interferometer, and
the first stage is capable of sandwiching the target between the first stage and the optical window.

6. The biological measurement device according to claim 5,
wherein the optical window protrudes from the second stage towards the first stage.

7. The biological measurement device according to any one of claims 1 to 6,
wherein the first stage is capable of deforming in response to a volume fluctuation caused by biological activity of the target.

8. The biological measurement device according to any one of claims 1 to 7,
wherein the target is a human body, and
the pressure of the gaseous body is 30 mmHg or greater and 80 mmHg or less.

9. The biological measurement device according to any one of claims 1 to 8,
wherein the gaseous body is air.

10. The biological measurement device according to any one of claims 1 to 9,
wherein the second stage is a jig including an upper plate, a lower plate and a plurality of support portions connecting the upper plate to the lower plate,
the second stage is disposed on a mechanical stage configured to move in three perpendicular directions in accordance with control signals of a controller,
the first stage is disposed on an upper surface of the lower plate of the second stage, and
the light transmitting plate protrudes from a lower surface of the upper plate of the second stage.

11. A biological measurement method for measuring a target by using a biological measurement device including a phase modulation type interferometer, a first stage, a light transmitting plate disposed between the first stage and the interferometer in a direction of an optical axis of measurement light of the interferometer, and a second stage supporting the light transmitting plate, the method comprising:
retaining the target with the first stage between the first stage and the light transmitting plate in the direction of the optical axis of the measurement light of the interferometer;
pressing the target retained in the first stage against the light transmitting plate with the first stage; and
measuring the target while the target is pressed against the light transmitting plate.
